# EUROPEAN PATENT SPECIFICATION

(11) **EP 3 946 262 B1**
(45) Date of publication and mention of the grant of the patent: **31.07.2024**
(21) Application number: 20764081.4
(22) Date of filing: 28.08.2020
(51) Int. Cl.: A61K 9/00, A61K 9/16, A61K 9/50, A61P 29/00, A23L 33/105

(54) **LOADED GRANULES, THEIR PROCESS OF PRODUCTION AND THEIR USES**
CANNABINOIDBELADENE GRANULATE, DEREN VERFAHREN ZUR HERSTELLUNG UND DEREN VERWENDUNGEN
GRANULÉS CHARGÉS DE CANNABINOÏDES, LEUR PROCÉDÉ DE PRODUCTION ET LEURS UTILISATIONS

(30) Priority: 30.08.2019 EP 19306056
(43) Date of publication of application: 09.02.2022
(60) Divisional application: 24158929.0
(73) Proprietor: EVIE SA, 1219 Chatelaine (CH)
(72) Inventor: SUID, Xavier, 44510 LE POULIGUEN (FR)
(74) Representative: Regimbeau
(86) International application number: PCT/EP2020/074062
(87) International publication number: WO 2021/038029

(56) References cited:
- DE-A1- 102014 213 548
- FR-A1- 2 967 066
- FR-A1- 2 967 066
- US-A1- 2018 147 179
- US-A1- 2018 344 786
- "Aulton's Pharmaceutics : The Design and Manufacture of Medicines", 30 June 2017, article PORTER STUART C.: "32 Coating of tablets and multiparticulates", pages: 593 - 593, XP093057198
- ERIC P BARON: "Comprehensive Review of Medicinal Marijuana, Cannabinoids, and Therapeutic Implications in Medicine and Headache: What a Long Strange Trip It's Been", HEADACHE, 1 January 2015 (2015-01-01), United States, pages 885 - 916, XP055730451, Retrieved from the Internet <URL:https://realmofcaring.org/wp-content/uploads/2019/10/Comprehensive-Review-of-Medicinal-Marijuana-Cannabinoids-and-Therapeutic-Implications-in-Medicine-and-Headache-What-a-Long-Strange-Trip-It's-Been.pdf> DOI: 10.1111/head.12570

## Description

### FIELD OF THE INVENTION

The present invention concerns orally-dispersible granules containing one or more active cannabinoid compounds, a process for producing said granules and uses of said granules as nutritional complements. The present invention also relates to said cannabinoid loaded granules for their use as a medicament.

### BACKGROUND OF THE INVENTION

The plant *Cannabis Sativa L.* (also called marijuana) produces phytocannabinoids, natural biochemical compounds characterized by their capacity to interact with the cannabinoid receptors in brain cells.

This plant has been known for thousands of years for its effect on the human body. Cannabinoids are largely consumed by smoking or vaporizing of dried cannabis plant material. These delivery systems are unhealthy, inconvenient and lack proper dosage control. Moreover, the consumed plant extract contains a combination of different phytocannabinoids that are all ingested.

However, it may be preferable to consume only some of these cannabinoid compounds, for example those that do not present any psychotropic effect.

About 110 different phytocannabinoids have been identified so far. Among them, the most abundant are tetrahydrocannabinol (THC), cannabidiol (CBD), and cannabinol (CBN), followed by cannabigerol (CBG), cannabichromene (CBC) and cannabinodiol (CBND).

The most notable cannabinoid is the trans-delta9-tetrahydrocannabinol (Δ9-THC), a powerful psychoactive compound that is responsible of euphoric and mind-altering effects. Another important phytocannabinoid is cannabidiol (CBD), a compound that is non-psychotropic but presents numerous pharmacological effects, including antipsychotic, analgesic, neuroprotective, anticonvulsant, antiemetic, antineoplasic, antiarthritic, antioxidant and anti-inflammatory effects.

Techniques for the extraction and isolation of cannabinoids have been developed in order to benefit of the advantageous effects of each cannabinoid individually, and notably of the cannabidiol that is free of psychoactive effects.

It has been clinically proven that cannabidiol administration has positive effects to alleviate neuropathic pain in individuals suffering from multiple sclerosis, and in cases of psychosis, movement disorders and anxiety behavorials, including generalized anxiety disorder (GAD), panic disorder (PD), post-traumatic stress disorder (PTSD), social anxiety disorder (SAD) and obsessive-compulsive disorder (OCD). These anxiety-related disorders constitute an immense social and economic burden (Blessing *et al.,* 2015).

Cannabidiol administration is well tolerated in humans, across a wide range dose, up to 1500 mg/day (orally), with no reported psychomotor slowing, negative mood effects, or vital sign abnormalities noted (Bergamaschi *et al.,* 2011).

The first FDA-approved drug containing CBD was Epidiolex^{®}, a liquid formulation of highly purified plant-derived cannabidiol, intended for the treatment of two rare, serious childhood epilepsy syndromes.

Outside of the United States, the drug Sativex^{®} comprising CBD and Δ9-THC in a 1:1 proportion is approved for the treatment of spasticity due to multiple sclerosis, in numerous countries. This drug is proposed under the form of an oromucosal spray.

Isolated phytocannabinoids may be consumed by ingestion, by inhalation or by transdermal delivery. Phytocannabinoids can be extracted from the plant with alcohols, and then applied in the oral cavity. They can also be extracted into oils and then administered orally, or via the nasal mucosa. Oily preparations may be proposed as such, or in the form of sprays, contained in gelatin capsules, or included in transdermal compositions.

Different galenic preparations comprising cannabinoids for therapeutic or non-therapeutic (recreational) uses, have been proposed:
- The patent US 9,095,563 describes topical compositions comprising *Cannabis species* extracts;
- The patent application WO 2017/189375 discloses chewing gum compositions comprising at least one isolated cannabinoid, nicotine, and at least one flavouring agent and one sweetening agent;
- The patent application WO 2017/208072 discloses nasal cannabidiol compositions, comprising an oily vehicle such as a vegetable oil;
- The patent application WO 2017/180707 relates to ingestible films comprising substances extracted from *Cannabis Sativa,* said films consisting of a matrix and an isolated cannabinoid in a concentration greater than 90%;
- The patent application WO 2017/185038 discloses CBD oral formulations comprising furthermore a compound intended for obtaining a fast-acting physiological effect of CBD;
- The patent application WO 2018/129097 relates to nutritional complements comprising synthetic cannabinoids in combination with N-acetylated fatty amino acids, which increase the water-solubility of cannabinoids;
- The patent US 10,434,084 describes a powder enriched in cannabinoids, made of tiny coated particles of sugars. This powder may be used as a dry premix for making a beverage or a cooked food;
- The patent application US 2018/344786 relates to an oily composition comprising a combination of cannabinoids and terpenes.

Studies of pharmacokinetics have shown that oral formulations provide the most favorable pharmacokinetic profile, when compared to transdermal applications (Bartner *et al.,* 2018).

Nevertheless, classical oral forms may have undesirable side effects, and in particular:
- Cannabinoid containing compositions may induce bad taste and/or dryness of the mouth;
- Administration of tablets and capsules requires the drinking of water, and some individuals may experience nuisance in swallowing bulky conventional dosage forms;
- Dry premix for beverages or food necessitate water and/or ingredients; they cannot be consumed as such;
- Oily formulations are likely to spill from their vials, and therefore their transport is complicated; moreover, this administration form lacks proper dosage control.

Culinary preparations containing cannabinoid(s), such as cakes and candies, have been proposed; but they are often loaded with very small amounts of cannabinoid, and due to their nature of perishable foods, are not stable over time.

It is an object of the present invention to furnish an oral formulation for the administration of at least one cannabinoid, presenting the following advantages:
- The oral formulation allows an easy control of the desired dosage;
- The oral formulation is pleasant to use;
- The oral formulation does not generate any bad taste in the mouth, it does not dry the buccal mucosa;
- The oral formulation does not need water for its administration; more generally, it does not necessitate any preparation, and can be administrated as it is, under any circumstance (for example while travelling).

The oral formulation advantageously comprises at least one terpene. Advantageously, the oral formulation according to the present invention is elegantly presented; its administration is pleasant; in consequence, this formulation is consumed in an enjoyable and playful manner. Furthermore, the consumption of this oral formulation is discreet.

### SUMMARY OF THE INVENTION

The present invention relates to cannabinoid loaded granules consisting of orodispersible sugar granules containing at least one cannabinoid compound, wherein each granule presents a diameter of 2 to 10 millimeters before loading, and wherein said cannabinoid loaded granules are intended to be administered sublingually.

In particular, said granules are composed of lactose, saccharose, xylitol, or a mix thereof.

In a preferred embodiment, said cannabinoid loaded granules further contain at least one terpene, preferably a combination of at least two terpenes.

In another aspect, the present invention relates to a process of production of cannabinoid loaded granules such as described above, comprising at least the following steps:
a) Addition to orodispersible sugar granules presenting a diameter of 2 to 10 millimeters of at least one cannabinoid compound,
b) Air-drying of the granules,
c) Repeating of said successive steps (a) and (b) at least twenty times,
d) Optionally, coating of the cannabinoid loaded granules with: a sugar syrup comprising a coloring or a flavoring agent, natural gum, natural wax, or any combination thereof.

The present invention also relates to said cannabinoid loaded granules for their use as a medicament.

The present invention also relates to said cannabinoid loaded granules for their use in the treatment and/or prevention of chronic pain, inflammatory disorders, behavioral disorders, and anxiety disorders.

In another aspect, the present invention relates to the use of cannabinoid loaded granules as a nutritional complement.

The present invention also concerns a kit for its use as a nutritional complement, comprising, in a single package, at least two dispensing devices comprising cannabinoid loaded granules further containing at least one terpene, wherein the at least two dispensing devices are distinct from each other in that said at least one terpene is different from one dispensing device to another.

### DETAILED DESCRIPTION OF EMBODIMENTS OF THE INVENTION

Unless stated otherwise, the following terms and phrases as used herein are intended to have the following meanings:
The term "about" hereby designates a range of more or less 10% of the indicated amount.

In the sense of the invention, "cannabinoid compound" and "cannabinoid" are used interchangeably and both designate a class of diverse chemical compounds that acts on cannabinoid receptors in cells. This class includes the endocannabinoids (produced naturally in the body by animals), the phytocannabinoids (found in cannabis and some other plants), and synthetic cannabinoids (manufactured artificially).

In the sense of the invention, the term "cannabinoid" includes all derived forms of cannabinoids, in particular those chemically derived from phyotocannabinoids.

The most notable phytocannabinoid is tetrahydrocannabinol (THC), the primary psychoactive compound in cannabis. Its chemical name is trans-Δ9-tetrahydrocannabinol, and its CAS number is 1972-08-3. THC presents the following developed chemical structure:

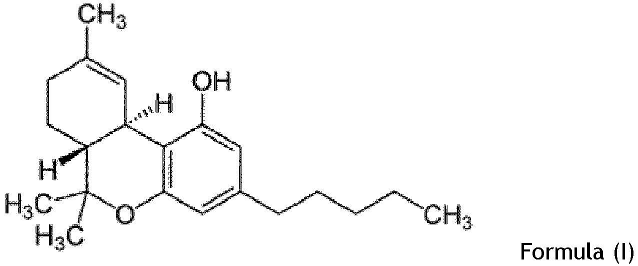

In the present application, the abbreviations Δ9-THC and THC are used indifferently, both designating trans-Δ9-tetrahydrocannabinol.

Cannabidiol (CBD) designates the compound referenced under CAS number 13956-29-1, presenting the following developed chemical structure:

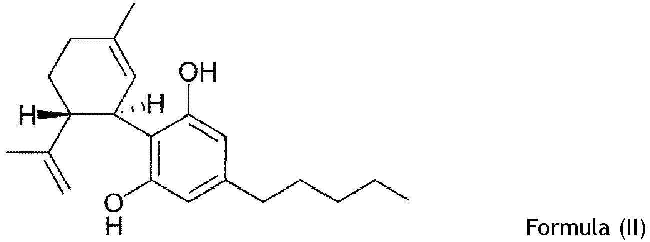

Initially discovered in 1940, it was isolated from the *Cannabis sativa* plant, wherein it represents up to 40% of the plant's extract.

Cannabidiol has a broad pharmacological profile, including interactions with several receptors, specifically the cannabinoid type 1 receptor (CB1R), the serotonin 5-HT1A receptor, and the transient receptor potential (TRP) vanilloid type 1 (TRPV1) receptor. In addition, CBD may also regulate, directly or indirectly, the peroxisome proliferator-activated receptor-γ, the orphan G-protein-coupled receptor 55, the equilibrative nucleoside transporter, the adenosine transporter, additional TRP channels, and glycine receptors.

Cannabidiol has very low affinity for the cannabinoid CB, and CB₂ receptors but is said to act as an indirect antagonist of these receptors.

Synthetic cannabinoids encompass all chemically synthetized compounds structurally related to THC and cannabidiol, as well as the nonclassical cannabinoids designated as cannabimimetics.

### Features of the granules

The present invention concerns cannabinoid loaded granules consisting of orodispersible sugar granules loaded with at least one cannabinoid compound wherein each granule presents a diameter of 2 to 10 millimeters before loading, and wherein said cannabinoid loaded granules are intended to be administered sublingually.

In the sense of the invention, the phrase "cannabinoid loaded granules" designate granules that have been loaded, by any technique known by the person skilled in the art, with at least one cannabinoid compound. Said loading techniques include:
- "impregnation", consisting of the penetration of a cannabinoid compound into the matrix of the granule (corresponding to "loading into"), and
- "sugar-coating", consisting of the agglomeration of a cannabinoid compound around the granule as an outer layer, corresponding to the coating of the granule with a sugar syrup and then application of said cannabinoid compound on the sugar syrup layer (corresponding to "loading onto").

The cannabinoid loaded granules of the present invention are granules containing cannabinoid(s), whatever the loading technique. In other words, the term "contain" as used herein to refer to the granules encompasses both the "load into" and "load onto" embodiments.

In the sense of the invention, the phrase "cannabinoid loaded granules" designate granules that have been loaded, by any technique known by the person skilled in the art, with at least one cannabinoid.

The orodispersible sugar granules, also called pellets or beads, are generally used as homeopathic medicine supports. Homeopathic pills are indeed made from granules made of an inert substance such as sugars, upon which a drop of liquid homeopathic preparation is placed and allowed to evaporate.

Neutral orodispersible sugar granules (i.e. without any active compound) are commercially available and well known by the person skilled in the art. In the sense of the invention, the phrase "neutral granules" designate granules before any step of loading.

In a specific embodiment of the invention, said orodispersible sugar neutral granules are composed of lactose, saccharose, xylitol, or a mix thereof.

Preferentially, the neutral granules are composed of 100% xylitol, a sugar alcohol of CAS number 87-99-0. Xylitol is industrially prepared from lignocellulose, issued from wood and agricultural waste.

These granules are orally dispersible (also defined as orodispersible, mouth dissolvable, melt-in-mouth or porous granules), which means that the granules are designed to get dispersed or to disintegrate when they contact saliva, without the aid of water, and then release the active compound(s) they contain.

These granules are intended for a sublingual administration of the active compounds they contain.

These granules are advantageously useful in conditions in which water is not available, or prohibited as before surgery.

The best time for an orodispersible granule to disintegrate into the mouth is considered to be less than a minute. Mostly, the disintegration time varies from 5 to 30 seconds.

Neutral granules used for producing cannabinoid loaded granules have preferably a circular shape, such as small beads or balls or spheres.

Preferably, neutral granules and loaded granules of the invention have a diameter superior to 1 millimeter.

According to the invention, the diameter of each neutral granule is of about 2 to 10 millimeters before loading, preferably of about 3 to 4 millimeters. In a specific embodiment of the invention, each granule presents a diameter of about 3 to 4 millimeters, in particular a diameter of 3.7 millimeters.

In a preferred aspect of the invention, each neutral granule weights about 30 to 300 milligrams, preferably about 30 to 150 milligrams. Typically, these neutral granules are commercially available under batches of "8 for one gram" (i.e. each granule weights about 125 mg), "10 for one gram" (each granule weights about 100 mg), "20 for one gram" (each granule weights about 50 mg), and "25 for one gram" (each granule weights about 40 mg).

### Cannabinoid compound

In a specific embodiment of the invention, the cannabinoid that is used for loading neutral granules is selected from the group consisting of: cannabidiol (CBD), cannabigerol (CBG), trans-Δ9-tetrahydrocannabidiol (THC), and a mix thereof.

According to a first embodiment, the granules contain cannabidiol (CBD). These granules are especially intended for individuals wishing to consume only cannabidiol, via a sublingual way of administration.

In particular, the granules contain a product such as PURE CBD, an isolate of cannabidiol.

According to a second embodiment, the granules contain trans-Δ9-tetrahydrocannabidiol (THC).

According to a third embodiment, the granules contain a mix of both cannabidiol (CBD) and trans-Δ9-tetrahydrocannabidiol (THC). This mix can be in any proportion for each component, for example 50% CBD and 50% THC, or 20% CBD and 80% THC or reciprocally, or 90% CBD and 10% THC or reciprocally.

In a specific embodiment, CBD and THC can be combined in a specific ratio, comprised between 99:1 of CBD/THC and 1:99 of CBD/THC, the limit values of this range being included.

CBD and THC have many overlapping physiological effects, although they work via different mechanisms of action. When combined, CBD and THC can enhance each other's benefits while reducing unwanted effects, including the psychoactive or impairing effects of THC.

According to a fourth embodiment, the granules contain cannabidiol (CBD), trans-Δ9-tetrahydrocannabidiol (THC), or a mix thereof, and a further other cannabinoid compound.

According to a fifth embodiment, the granules contain cannabidiol (CBD) in combination with cannabigerol (CBG).

The at least one cannabinoid used for loading the neutral granules may be of natural origin or synthetic.

Preferably, the at least one cannabinoid is a phytocannabinoid of natural origin. In particular, the at least one cannabinoid may be a phytocannabinoid extracted from the plant *Cannabis sativa.*

Preferably, the at least one cannabinoid will be under a purified form comprising at least 90% of said cannabinoid in weight. This purified form might be in particular an isolate of said cannabinoid.

Extracts from the plant *Cannabis sativa* might be under different forms such as emulsion, oil, paste, liquid, resin, crystal, powder, or pulp.

These different forms of cannabinoid extract are classified in two main categories: "full spectrum" or "isolate". The "full spectrum" contains mainly one cannabinoid, but also small amounts of other cannabinoids from the plant.

The "isolate" consists of the purified cannabinoid that has been extracted from the plant and isolated from the other cannabinoids.

According to a preferred embodiment of the invention, said isolate contains at least 95%, preferably at least 99% and more preferably about 99.9% in weight of the considered cannabinoid, compared to the total weight of the isolate.

Cannabidiol isolates with more than 99% of CBD are commercially available, for example at SPECTRUMS Europe or at FOLIUM BIOSCIENCES (US).

These isolates, obtained from refining of oily full spectrum extracts, are usually under the form of a crystalline powder. This powder is soluble in oil but not in water. Interestingly, these isolates do not present any taste nor flavor. Advantageously, said CBD isolate does contain only traces of THC, or better does not contain any THC.

### Sugar coating of the granules

In a preferred embodiment of the invention, the granules are sugar coated with a sugar syrup and an extract containing at least one cannabinoid compound.

This technique called "sugar coating" of granules is a routine procedure for the person skilled in the art of homeopathic granules, as well as for the person skilled in the art of confectionary. This technique is advantageous since it allows a uniform repartition of the at least one cannabinoid compound into an external layer of sugar made around the granule.

It is typically performed using a coating turbine. Coating turbines useful for this step are in particular those of type DRIAM, GLATT or MANESTY.

The process of sugar coating comprises three steps: coating of the granules with a sugar syrup, then application onto the granules of an extract containing at least one cannabinoid compound (full-spectrum or isolate, preferentially an isolate), and finally air-drying of the coated granules.

The sugar syrup consists of water and at least one sugar, such as a polyol, a monosaccharide, a disaccharide, or any combination thereof. Preferably the sugars are chosen among lactose, saccharose, a polyol or a mix thereof. In particular the sugar is a polyol, and preferably is xylitol.

In a preferred embodiment of the invention, the sugar syrup is in a concentration of about "60 BRIX", corresponding to 60 grams of sugar dissolved in 100 ml of water. The sugar syrup might also be a solution at 40, 50, 65, 70, 80 or 90 BRIX.

Advantageously, the extract containing at least one cannabinoid compound is derived from the plant *Cannabis Sativa L.*

Preferably it is an isolate comprising only one cannabinoid compound.

In a preferred embodiment, this isolate comprises at least 95% by weight of said one cannabinoid compound.

### Dose of the loaded cannabinoid

Dosage is the key factor in achieving the most benefits and least adverse effects of *Cannabis sativa* extracts. Although variable from one individual to another, optimal (i.e. safe, with no side effects) mean day doses of cannabinoid for adults have been established by physicians, and are the following:
- For CBD, 25 to 30 milligrams per day; and
- For THC, 20 to 30 milligrams per day.

Preferably, each single dose of administration shall not exceed 10 mg.

In a specific embodiment, the cannabinoid loaded granules are prepared to contain a specific amount of at least one cannabinoid or a mix thereof.

For example, each granule may contain 5, 10, 15, 20, 25 or 30 mg of cannabinoids, i.e. of one single cannabinoid or of a mix of at least two cannabinoid compounds.

Preferably, each granule contains a dose of one cannabinoid from about 2 mg to about 8 mg, preferentially from about 4 mg to about 6 mg, and more preferably a dose equal to about 5 mg.

In a specific embodiment of the invention, one cannabinoid loaded granule weights about 130 mg, including 125 mg of sugar granule and 5 mg of the at least one cannabinoid. In this embodiment, the at least one cannabinoid represents 3.85 % of the total dry weight of the loaded granule.

In another specific embodiment of the invention, one cannabinoid loaded granule weights about 45 mg, including 40 mg of sugar granule and 5 mg of the at least one cannabinoid. In this embodiment, the at least one cannabinoid represents 11.11 % of the total dry weight of the loaded granule.

In preferred embodiments of the invention, the at least one cannabinoid represents about 2 % to 15% in weight of the total dry weight of the cannabinoid loaded granule.

### Cannabinoid loaded granules further containing terpenes

In a specific embodiment of the invention, the cannabinoid loaded granules further contain at least one terpene, preferably a combination of at least two terpenes.

More specifically, the invention concerns cannabinoid loaded granules further containing at least one terpene, preferably a combination of at least two terpenes.

Advantageously, the cannabinoid loaded granules of the invention are impregnated with a solution comprising a combination of at least two terpenes.

Terpenes are a class of organic hydrocarbons that are produced by a variety of plants, particularly conifers, and by some insects. Terpenes and their derivative terpenoids are the primary constituents of the essential oils. Terpenes are also major constituents of *Cannabis sativa* plants, which contain at least 120 identified compounds.

Terpenes have desirable properties for use in food and pharmaceutical industries. While terpenes and terpenoids occur widely, their extraction from natural sources is often problematic. Consequently, they are often supplied as synthetic products issued from chemical synthesis.

In a preferred embodiment of the invention, the granules are impregnated with a solution comprising at least one terpene chosen among the following group: myrcene, d-limonene, alpha-pinene, linalol, borneol, carophyllene, terpinolene, menthol, geraniol, bisabolol, beta-caryophyllene, humulene, linalool, farnesene, a-phellandrene, and any combination thereof.

Impregnation of granules is a routine procedure for the person skilled in the art of homeopathic granules. The procedure has been extensively described in literature, for example in US patent 4,703,717. This technique comprises the use of:
- a liquid comprising the active compounds (at least one terpene),
- porous sugar granules of a desired size/weight; and
- an impregnating device, with control means.

Impregnating devices are commercially available, in any homeopathic devices shop, such as for example at http://www.vanda-france.fr/.

Advantageously, the granules of the invention are impregnated with terpene(s) at a rate of 0.2 % to 2 %, preferably at a rate of impregnation of 0.5 %.

As presented in the examples section, a common dose is about 25 grams of terpenes for 5000 grams of xylitol granules, corresponding to an impregnation rate of 0.5%.

In a specific implementation of the process of production of cannabinoid loaded granules, the granules are firstly impregnated with a dynamized solution comprising at least one terpene, and then are loaded with cannabinoid(s).

Optionally, loaded granules can be coated with a natural gum, for example with arabic gum, right after the step of terpenes impregnation. This optional step is useful to fix and isolate the at least one terpene, and further helps to solidify the granule.

### Additional compounds

Cannabinoid loaded granules according to the invention may also comprise at least one additional active compound.

These active compounds may be chosen in particular among mushroom extracts, caffeine, flavonoids, and combinations thereof.

Mushroom extracts are in particular extracts from the following mushrooms:
- *Hydnum repandum,* commonly known as the sweet tooth, wood hedgehog or hedgehog mushroom, a Basidiomycete fungus of the family Hydnaceae;
- *Hericium erinaceus,* also called lion's mane mushroom, monkey head mushroom, bearded tooth mushroom, satyr's beard, bearded hedgehog mushroom, pom pom mushroom, or bearded tooth fungus, belonging to the tooth fungus group;
- chaga, i.e. *Inonotus obliquus,* from the family Hymenochaetaceae ;
- reishi, also known as Lingzhi or Ganoderma lingzhi, a polypore fungus belonging to the genus Ganoderma;
- *Trametes versicolor,* also known as Coriolus versicolor or Polyporus versicolor, a polypore mushroom.

In a specific embodiment, cannabinoid loaded granules according to the invention further comprise at least one mushroom extract.

In a specific embodiment, cannabinoid loaded granules according to the invention further comprise caffeine.

In a specific embodiment, cannabinoid loaded granules according to the invention further comprise at least one flavonoid.

The cannabinoid loaded granules may further contain additional compounds selected from the group of solubilizers, thickeners, surfactants, coloring agents (in particular for whitening the granules), flavoring agents, effervescent agents, antioxidants, bioadhesive agents, pH modifying agents, vitamins, minerals, permeability or penetration enhancers, absorption enhancers, serotonin, caffeine, amino acids, and mixtures thereof.

Permeability or penetration enhancers and absorption enhancers, if present, are added in order to improve the absorption of the cannabinoid by the mucosal tissues of an individual.

Vitamins are in particular selected from the group consisting of thiamin, riboflavin, nicotinic acid, pantothenic acid, pyridoxine, biotin, folic acid, vitamin B12, lipoic acid, ascorbic acid, vitamin A, vitamin D, vitamin E, and vitamin K.

These additional compounds may be added to the granules by impregnation or as part of the sugar-coating of the granules.

### Final coating of the cannabinoid loaded granules

Final coating of the loaded granules enables an aesthetic appeal, while improving the stability of the granules. In particular, if traces of crystalline powder of the cannabinoid isolate are still present around the loaded granules, the final coating might allow the fixation of said powder traces onto the granule.

In a specific embodiment of the invention, the cannabinoid loaded granules are coated with: a sugar syrup comprising a coloring or flavoring agent, natural gum, natural wax, or any combination thereof.

This optional step allows the obtention of bright granules. Further, this final coating protects them from breakage.

The technique for obtaining this coating has been described above.

This final coating is an outer layer on the whole surface of the granule, of a thickness inferior to 10 microns. It might comprise notably:
- A natural gum such as arabic gum or xanthan gum; or
- A natural wax such as carnauba wax or beeswax; or
- In the case of a sugar coating :
   ∘ A polyol such as xylitol; or
   ∘ A monosaccharide such as glucose or fructose; or
   ∘ A disaccharide such as saccharose, lactose, or sucrose, or
- Any combination thereof.

Optionally, the final coating layer may comprise coloring and/or flavoring agents.

The final coating layer may comprise a coloring agent chosen from appropriate synthetic or natural coloring agents, well known by the person skilled in the art.

The final coating layer may also contain flavoring agents chosen from synthetic flavor oils and flavoring aromatics and/or natural oils. Such flavoring agents might be chosen among those having one of the following flavors : mint, ginger, anise, cinnamon, peppermint, licorice, honey, vanilla, citrus oil, including lemon, orange, grape, lime and grapefruit, and fruit essences, including apple, pear, peach, strawberry, raspberry, cherry, plum, pineapple, apricot and so forth.

### Process of production of cannabinoid loaded granules

The present invention also concerns a process of production of cannabinoid loaded granules such as described above, comprising at least the following steps:
a) Addition to orodispersible sugar granules presenting a diameter of 2 to 10 millimeters of at least one cannabinoid compound,
b) Air-drying of the granules,
c) Repeating of said successive steps (a) and (b) at least twenty times,
d) Optionally, coating of the cannabinoid loaded granules with: a sugar syrup comprising a coloring or a flavoring agent, natural gum, natural wax, or any combination thereof.

The step of "addition to orodispersible sugar granules" can be performed by any technique known by the person skilled in the art. In particular, this step of addition of at least one cannabidiol compound might be:
- A step of application of an extract containing at least one cannabinoid compound, onto the orodispersible sugar granules; or
- A step of integration of at least one cannabinoid compound into the orodispersible sugar granules.

Optional steps can be added, at any time during the process described above: for example, a step of terpene(s) impregnation, followed by a step of scrubbing, can be implemented before or after the cannabinoid loading step (a).

In a particular embodiment of the invention, the process of production of cannabinoid loaded granules such as described above, comprises at least the following steps:
a1) Coating of orodispersible sugar granules with a sugar syrup, in particular a xylitol syrup,
a2) Application onto the xylitol-coated granules of an extract containing at least one cannabinoid compound,
b) Air-drying of the granules,
c) Repeating of said successive steps (a1), (a2) and (b), at least twenty times,
d) Optionally, further coating of the cannabinoid loaded granules with: a sugar syrup comprising a coloring or a flavoring agent, natural gum, natural wax, or any combination thereof.

In step (a1), orodispersible sugar granules are sprayed with a sugar syrup such as a xylitol syrup. These granules might have been previously impregnated with a solution comprising at least one terpene, preferably a combination of at least two terpenes.

Steps (a1), (a2) and (b) are performed in a coating turbine. These steps are realized successively and are repeated at least twenty times, preferably at least thirty times, more preferably at least forty times, and in a preferred manner about fifty times.

Step (d) consists of the final sugar-coating of the cannabinoid loaded granules, with a thin outer layer on the whole surface of the granule, comprising:
- A natural gum such as arabic gum or xanthan gum; or
- A natural wax such as carnauba wax or beeswax; or
- A sugar coating comprising water and :
   ∘ A polyol such as xylitol; or
   ∘ A monosaccharide such as glucose or fructose; or
   ∘ A disaccharide such as saccharose, lactose, or sucrose, and
   ∘ Optionally, a flavoring agent and/or a coloring agent, or
- Any combination thereof.

This step (d) can be repeated at least twice, for example:
- A first sugar-coating step is realized in order to cover the granules with a flavoring agent;
- A second coating step is realized with wax, for obtaining a bright appearance of the cannabinoid loaded granule.

The first sugar-coating step for covering the granules with a flavoring agent can be repeated at least twice, at least five times, at least ten, twenty or more times, before performing the second coating step.

Said coating is performed with a coating turbine. Coating turbines useful for this step are in particular those of type DRIAM, GLATT or MANESTY.

Another implementation of the process according to the invention is presented in example 4. This specific process comprises the following steps:
1) Impregnation of orodispersible sugar granules with at least one terpene;
2) Scrubbing with a natural gum;
3) Loading granules with at least one cannabinoid compound;
4) Coating of the granules with a sugar syrup, optionally comprising a flavoring agent;
5) Final coating of the granules with wax, for protection and brightness.

The present description also disclosesa process of production of cannabinoid loaded granules, comprising at least the following steps:
a) Lyophilization of a solution comprising at least one cannabinoid compound and one sugar,
b) Formation of granules from the lyophilized powder, for example using a tablet press,
c) Optionally, coating of the cannabinoid loaded granules with: a sugar syrup comprising a coloring or a flavoring agent, natural gum, natural wax, or any combination thereof.

This process is particularly useful in the case of the at least one cannabinoid compound is THC. This process is illustrated in the reference example 5.

### Uses of the cannabinoid loaded granules of the invention

The references to methods of treatment in the subsequent paragraphs of this description are to be interpreted as references to the compounds, pharmaceutical compositions and medicaments of the present invention for use in a method for treatment of the human or animal body by therapy.

The present invention also concerns cannabinoid loaded granules as described above, or as obtained by anyone of the processes as described above, for their use as a medicament.

Cannabinoid loaded granules are intended to be administered sublingually. Interestingly, this delivery route of cannabinoid(s) allows a rapid onset of the cannabinoid(s) through the oral mucosa membranes.

In another aspect, the invention concerns said cannabinoid loaded granules for their use in the treatment and/or prevention of chronic pain, inflammatory disorders, behavioral disorders, and anxiety disorders.

As used herein, the terms "treat", "treating" or "treatment" refers to the administration of therapy to an individual in an attempt to reduce the frequency and/or severity of symptoms of a disease, defect, disorder, or adverse condition of said individual.

As used herein, the terms "prevent", "preventing" or "prevention" refers to the administration of a therapeutic compound to an individual in an attempt to reduce the likelihood for said individual to develop a specific disease.

### Nutritional complement and kit comprising it

The present invention also relates to the use of cannabinoid loaded granules as described above, or as obtained by anyone of the processes as described above, as a nutritional complement.

As used herein, the term "nutritional complement" designates any dietary complement that comes further to the normal dietary regimen that is intended to provide substances that are not usually consumed.

Individuals consuming said nutritional complement may be "recreational users" of cannabinoids.

The present invention also relates to a kit for its use as a nutritional complement, comprising, in a single package, at least two dispensing devices comprising cannabinoid loaded granules further containing at least one terpene, wherein the at least two dispensing devices are distinct from each other in that said at least one terpene is different from one dispensing device to another.

In a particular embodiment, said kit further comprises means for communicating information or instructions for the use of said kit.

In a particular embodiment, said kit comprises cannabidiol loaded granules.

As already stated, an optimal daily dose of a cannabinoid, such as cannabidiol (CBD), is of about 25 mg a day, and consequently, in a specific embodiment, the optimal daily dose is of 5 granules containing 5 mg of CBD each.

According to this last embodiment, the user of the kit will choose 5 granules to consume during the day, according to his/her specific needs: concentration, sleepiness, fatigue, energy-load before exercising, etc.

When the kit contains three dispensing devices, the user might choose to consume two granules in the morning from one dispensing device, one granule at noon from another dispensing device, and two granules before sleeping at night from the last dispensing device.

Advantageously, the kit of the invention comprises enough granules for administration over one week (35 cannabinoid loaded granules), two weeks (70 cannabinoid loaded granules) or even one month (more than 140 cannabinoid loaded granules), the number of granules being based on the optimal daily dose of 5 granules a day.

These granules are distributed in at least two dispensing devices, preferably in three, four, five, six or seven dispensing devices.

Each dispensing device comprises about 5, 10, 15, 20, 25 or 30 cannabinoid loaded granules.

### EXAMPLES

Although the present invention herein has been described with reference to particular embodiments, it is to be understood that these embodiments are merely illustrative of the principles and applications of the present invention.

### Example 1. Process of preparation of cannabinoid loaded granules

In this experiment, xylitol granules have been loaded with 5 mg of CBD per granule. Used xylitol beads are about 3.7 mm in diameter and 0.04 g in weight.

### A - Impregnation of the granules with a combination of terpenes.

A solution comprising a combination of pure terpenes (Myrcene type) such as limonene, A-pinene, linalol, carophyllene, was used in the following proportion:
- 1 kg of xylitol beads, and
- 5 g of the terpenes solution.

Dynamization of the solution was repeated three times: during 20 seconds, 300 succussions. As well known by the person skilled in the art, liquid containing the active components are preferentially "dynamized" before impregnation. In homeopathy, "dynamization" refers to the vigorous shaking of an alcoholic solution comprising an active compound, in a process called "succussion".

Impregnation is realized either with an impregnator or with a coating turbine.
By impregnator:
   Supply of terpenes solution by addition to the beads
   Mixing: 30 min
   Drying time: 20 to 30 min
By turbine:
   Supply of terpenes solution by spraying (atomization with an automatic spray gun) the beads;
   Mixing: 30 min;
   Drying time: 20 to 30 min (with mini central air treatment); the air temperature is below 40°C.

At the end of this process, xylitol beads are impregnated with a combination of at least two terpenes (in this case, 5 g of terpenes for 1 kg of beads, i.e. 0.5% terpenes by weight per bead).

### B - Coating of xylitol beads impregnated with terpenes with xylitol syrup and then application of CBD or any other cannabinoid compound

In this example, 2 kg of xylitol beads (i.e. about 50 000 beads) impregnated with terpenes were used, with 250 g of cannabidiol (CBD), in order to dispense about 5 mg of CBD to each bead.

First, a xylitol syrup at 60 BRIX was prepared with 450 g of xylitol and 300 g of water, heated up to 80° C, then the temperature of the syrup is decreased between 50°C and 60° C, and the concentration of xylitol is adjusted to 60 BRIX.

Once the 60 BRIX syrup is obtained, the xylitol beads are introduced into the turbine for the coating.

Each cycle of coating and application of CBD comprises the following substeps:
Beads are sprayed with xylitol syrup, with an automatic spray gun, and then mixed for about 30 seconds;
CBD isolate is applied onto beads; and
Beads are dried with air treatment for 3 to 4 minutes, with a "cold" air at a temperature of about 25 °C.

These successive steps are repeated about 64 times.

The number of sprays depends on the cycle:
- For the first ten cycles, 8 sprays of xylitol syrup / CBD isolate are performed; during which 4.5 g of xylitol syrup (0.04 litre) and 2.25 g of cannabidiol are loaded onto the beads;
- During the cycles n°11 to 20, 12 sprays of xylitol syrup / CBD isolate are performed; 6.75 g of xylitol syrup and 3.375 g of cannabidiol are loaded onto the beads;
- From the cycle n° 21, 16 sprays of xylitol syrup / CBD isolate are performed, during which 4.5 g of cannabidiol are loaded.

With this process, the 250 g of cannabidiol are progressively introduced onto the 50 000 beads.

### Example 2: Further coating of cannabinoid loaded granules for flavour, colour and/or brightness

Advantageously, two steps of "final sugar-coating" are performed:
1. Coating of xylitol syrup with vanilla flavour (or any other aroma):
   Beads are sprayed with xylitol syrup with an automatic spray gun, and mixed for about 4 minutes;
   Beads are dried with air treatment, for about 3 minutes;
   During the last spray, vanilla flavour (2 g/kg) is added to the beads, with minimal air;
   Beads are dried.

This step is repeated about 25 times.

2. Carnauba wax is mixed with the CBD loaded granules, without any air, in the following proportion: 500 mg of wax for 1 kg of loaded granules.

### Example 3. Preparation of a kit

A kit comprising xylitol granules loaded with 5 mg CBD per granule, and other active compounds, comprises eight dispensing devices.

Each dispensing device comprises CBD-loaded granules comprising furthermore a mix of terpenes, said dispensing devices being distinct from each other in that said mix of terpenes is different from one dispensing device to another. In this example, each granule comprises 0.5% terpenes mix by weight.

Each one of the eight dispensing devices is designed for a specific use:
1) BRAIN FOCUS - "FOCUS"
   CBD-loaded granules comprise furthermore a combination of at least two terpenes chosen among: alpha-pinene, d-limonene, borneol and myrcene.
2) SPORT BOOSTER - "SPORT"
   CBD-loaded granules comprise furthermore a combination of of at least two terpenes chosen among: alpha-pinene, d-limonene, terpinolene, menthol, geraniol and bisabolol.
3) SLEEP
   CBD-loaded granules comprise furthermore a combination of at least two terpenes chosen among: alpha-pinene, beta-Caryophyllene, Humulene, linalool, and myrcene.
4) PAIN RELIEF
   CBD-loaded granules comprise furthermore a combination of at least two terpenes chosen among: alpha-pinene, d-limonene, geraniol, humulene, farnesene, linalool and myrcene.
5) SAFEGUARD (Immune system booster) - "IMMUNITY"
   CBD-loaded granules comprise furthermore a combination of at least two terpenes chosen among: alpha-pinene, d-limonene, beta-caryophyllene, a-phellandrene, linalool and myrcene.
6) APHRODISIAC STIMULANT (sensitive) - "INTIMACY"
   CBD-loaded granules comprise furthermore a combination of at least two terpenes chosen among: alpha-pinene, d-limonene, terpinolene, beta-caryophyllene, humulene, farnesene, linalool and myrcene.
7) RELAX
   CBD-loaded granules comprise furthermore a combination of at least two terpenes chosen among: farnesene, beta-caryophyllene, myrcene.
8) "PURE CBD"
   CBD-loaded granules with a combination of at least two terpenes. CBD is said "pure" since these granules do not contain any cannabigerol (CBG).

### Example 4. Illustration of an industrial process of preparation of loaded granules

### Phase 1: IMPREGNATION

### STEP 1 - CONTROL

Air temperature: between 20°- 30°
Hygrometry: between 20% and 35%

### STEP 2 - DEVICE START UP

Granules made of xylitol (5000 g) are deposited into the coating device.

The device is turned on. Mixing speed is comprised between 30 and 40 rpm (revolutions per minute).

### STEP 3 - DYNAMISATION OF THE SOLUTION TERPENES / ALCOHOL

The following compounds are mixed:
- natural terpenes (about 25g)
- ethylic alcohol (about 75g)

Terpenes and alcohol are mixed with dynamization: 2 to 6 times / between 300 and 500 succussions.

The dynamized solution is integrated in 4 steps into the coating device, and then:
- brewing for 10 to 30 minutes
- drying for 30 to 60 minutes, at a temperature comprised between 40° and 60°C.

### Phase 2: SCRUBBING

### STEP 1 - PRÉPARATION OF PRIMARY COMPOUNDS (dilution between 2% and 5%)

- Xylitol powder: about 100g
- Distilled water: 25g to 30g
- Arabic gum: 25g to 30g

### STEP 2 - PREPARATION OF THE GUM

For about 100g of dry powder:
- Arabic gum: 20g to 30g
- xylitol powder: 70g to 80g

Blend xylitol powder and arabic gum.

For about 50ml of solution:
- Arabic gum: 5g
- xylitol powder: 20g
- distilled water : 25g

Blend xylitol powder and arabic gum
Heat water up to 30° - 45° C
Mix the powder with water and maintain the temperature
Pour the mix into the coating device: stirring for 1 to 5 minutes.
Pour 100g of dry powder into the coating device
Brewing for about 1 min to 5 min
Drying between 20° et 30°C for about 1 hour.

### Phase 3: CBD- CBG COATING

### STEP 1 - RAW MATERIALS PREPARATION

Xylitol powder: between 2700g et 3500g
Distilled water: between 1000g et 1700g
CBD - CBG: about 1400g

### STEP 2 - XYLITOL SYRUP PREPARATION

- Pour distilled water into a hot beverage machine
- Adjust temperature between 65° and 85° C
- Pour xylitol powder
- Check the « Brix » syrup: between 60 and 90

### STEP 3 - CBD - CBG COATING

This coating process comprises between 40 et 70 steps. Each step consists of:
- pour into the coating device about 100 ml of xylitol syrup and 20 g of CBD-CBG
- mix for about 1 to 5 minutes
- dry between 20° et 30°C.

### Phase 4: XYLITOL SYRUP COATING

### STEP 1 - RAW MATERIALS PREPARATION

Xylitol powder: between 1250 and 1900g
distilled water: between 500 and 850 g
vanilla flavor: between 5g and 12g

### STEP 2 - XYLITOL SYRUP PREPARATION

- Pour distilled water into a hot beverage machine
- Adjust temperature between 65° and 85° C
- Pour xylitol powder
- Check the « Brix » syrup: between 60 and 90

### STEP 3 - XYLITOL SYRUP COATING

### Refill granules into the coating device

This coating process comprises between 10 et 30 steps. Each step consists of the following substeps:
- pour into the coating device about 50 ml of xylitol syrup • mix for about 1 to 5 minutes • dry between 20° et 30°C
- integrate the vanilla flavor between the 15th and 25th step

### Phase 5: PROTECTION - BRIGHTNESS

### STEP 1 - RAW MATERIAL PRÉPARATION

Carnauba wax: between 0,9 g and 2,5 g (for 5000 g of granules)

### STEP 2 - PROTECTION - BRIGHNESS

• Refill the granules into the coating device
• Pour carnauba wax into the coating device
• Brew for about 30 to 60 minutes
• Dry for about 30 minutes at a temperature of 20° to 30° C.

### Reference

### Example 5. Illustration of another industrial process of preparation of THC loaded granules

This process comprises the following steps:

### 1. Preparation of solution (1), comprising:

- THC (80% concentrated cannabis oil)
- Food grade ethyl alcohol
- terpenes

- Incorporate the THC oil into the ethanol;
- Incorporate the terpenes into the THC and ethanol solution;
- The solution is then homogenized by dynamization

### 2. Preparation of a solution (2) made of sugar and water

- Dilution of a polyol and lecithin in water;
- Heating of the water between 70 and 75° Celsius;
- Incorporation of xylitol into water;
- Incorporation of lecithin into water;
- The solution is homogenized by dynamization.

### 3. Incorporation of solution 2 into solution 1:

- The final solution is homogenized by ultrasonic homogenization;
- The operation is repeated up to the obtention of particles of a size between 100 and 200 microns.

### 4. Lyophilization of the solution:

The solution is freeze-dried to produce a powder by the following technique:
- The solution is dispensed into the trays
- Incubation in the freezer dryer:
   ∘ 9 to 12 hours of freezing
   ∘ 24 to 28 hours of lyophilization

### 5. Preparation and homogenization of the powder for incorporation into a tablet press.

The lyophilized powder is mixed with an excipient (Mannitol, xylitol....) in the following proportions:
- 43% Lyophilized powder
- 57% excipient

Powder homogenization is carried out by dry granulation, hammer granulator or wheel granulator. Particles of a size between 100 and 200 microns are obtained.

### 6. Powder shaping

Granules of diameter comprised between 3 to 4 mm are formed using the tablet press.

### 7. First coating of the granules

The coating process consists of between 10 and 30 steps.

Each step consists of the following substeps:
∘ Addition of about 50 ml of Xylitol syrup (69% Xylitol - 31% water) into the coating machine;
∘ Stirring between 1 and 5 minutes;
∘ Drying between 20° and 30° Celsius

### 8. Second coating of the granules for protection, gloss

- Granules are refill into the coating machine;
- Addition of carnauba wax on the granules;
- Brewing between 30 minutes and 1 hour.

### REFERENCES

### PATENTS

US 9,095,563
WO 2017/189375
WO 2017/208072
WO 2017/180707
WO 2017/185038
WO 2018/129097
US 10,434,084
US 2018/344786

### BIBLIOGRAPHIC REFERENCES

Blessing EM, Steenkamp MM, Manzanares J, Marmar CR. Cannabidiol as a Potential Treatment for Anxiety Disorders. Neurotherapeutics. 2015 Oct;12(4):825-36.
Bergamaschi MM, Queiroz RH, Zuardi AW, Crippa JA. Safety and side effects of cannabidiol, a Cannabis sativa constituent. Curr Drug Saf. 2011 Sep 1;6(4):237-49.
Bartner LR, McGrath S, Rao S, Hyatt LK, Wittenburg LA. Pharmacokinetics of cannabidiol administered by 3 delivery methods at 2 different dosages to healthy dogs. Can J Vet Res. 2018 Jul;82(3):178-183.

## Claims

1. Cannabinoid loaded granules consisting of orodispersible sugar granules loaded with at least one cannabinoid compound, wherein each granule presents a diameter of 2 to 10 millimeters before loading,
and wherein said cannabinoid loaded granules are intended to be administered sublingually.

2. Cannabinoid loaded granules according to claim 1, wherein said orodispersible sugar granules are composed of lactose, saccharose, xylitol, or a mix thereof.

3. Cannabinoid loaded granules according to claim 1 or 2, wherein each granule presents a diameter of 3 to 4 millimeters, more or less 10%, and/or weights 30 to 150 milligrams, more or less 10%.

4. Cannabinoid loaded granules according to any one of claims 1 to 3, wherein said at least one cannabinoid compound is selected from the group consisting of: cannabidiol (CBD), cannabigerol, trans-Δ9-tetrahydrocannabidiol (THC), and a mix thereof.

5. Cannabinoid loaded granules according to any one of claims 1 to 4, wherein said at least one cannabinoid compound is synthetic, or is extracted from the plant *Cannabis sativa* and is under the form of an isolate containing at least 95% in weight of said cannabinoid compound, compared to the total weight of the isolate.

6. Cannabinoid loaded granules according to any one of claims 1 to 5, wherein said granules are sugar coated with a sugar syrup and an extract containing at least one cannabinoid compound.

7. Cannabinoid loaded granules according to any one of claims 1 to 6, wherein each cannabinoid loaded granule contains a dose of at least one cannabinoid compound from 2 mg to 8 mg, more or less 10%, and preferentially contains a dose equal to 5 mg, more or less 10%.

8. Cannabinoid loaded granules according to anyone of claims 1 to 7, wherein said granules further contain at least one terpene, preferably a combination of at least two terpenes.

9. Cannabinoid loaded granules according to anyone of claims 1 to 8, wherein said granules are further coated with: a sugar syrup comprising a coloring or flavoring agent, natural gum, natural wax or any combination thereof.

10. Process of production of cannabinoid loaded granules according to any one of claims 1 to 9, comprising at least the following steps:
a) Addition to orodispersible sugar granules presenting a diameter of 2 to 10 millimeters of at least one cannabinoid compound,
b) Air-drying of the granules,
c) Repeating of said successive steps (a) and (b) at least twenty times,
d) Optionally, coating of the cannabinoid loaded granules with: a sugar syrup comprising a coloring or a flavoring agent, natural gum, natural wax, or any combination thereof.

11. Cannabinoid loaded granules according to any one of claims 1 to 9, or obtained according to the process of claim 10, for their use as a medicament.

12. Cannabinoid loaded granules according to any one of claims 1 to 9, or obtained according to the process of claim 10, for their use in the treatment and/or prevention of chronic pain, inflammatory disorders, behavourial disorders, and anxiety disorders.

13. Use of cannabinoid loaded granules according to any one of claims 1 to 9 or obtained according to the process of claim 10, as a nutritional complement.

14. Kit for its use as a nutritional complement, comprising, in a single package, at least two dispensing devices comprising cannabinoid loaded granules further containing at least one terpene according to claim 8, wherein the at least two dispensing devices are distinct from each other in that said at least one terpene is different from one dispensing device to another.

## Patentansprüche

1. Cannabinoidbeladene Granulate, die aus orodispersiblen Zuckergranulaten bestehen, die mit mindestens einer Cannabinoidverbindung beladen sind, wobei jedes Granulum vor dem Beladen einen Durchmesser von 2 bis 10 Millimetern aufweist,
und wobei die cannabinoidbeladenen Granulate dazu bestimmt sind, sublingual verabreicht zu werden.

2. Cannabinoidbeladene Granulate nach Anspruch 1, wobei die orodispersiblen Zuckergranulate aus Lactose, Saccharose, Xylitol oder einem Gemisch davon bestehen.

3. Cannabinoidbeladene Granulate nach Anspruch 1 oder 2, wobei jedes Granulum einen Durchmesser von 3 bis 4 Millimetern, plus oder minus 10%, aufweist und/oder 30 bis 150 Milligramm, plus oder minus 10%, wiegt.

4. Cannabinoidbeladene Granulate nach einem der Ansprüche 1 bis 3, wobei die mindestens eine Cannabinoidverbindung ausgewählt ist aus der Gruppe, die besteht aus: Cannabidiol (CBD), Cannabigerol, Trans- Δ9-Tetrahydrocannabidiol (THC) und einem Gemisch davon.

5. Cannabinoidbeladene Granulate nach einem der Ansprüche 1 bis 4, wobei die mindestens eine Cannabinoidverbindung synthetisch ist, oder von der Pflanze *Cannabis sativa* extrahiert ist und in der Form eines Isolats vorliegt, das, bezogen auf das Gesamtgewicht des Isolats, mindestens 95 Gew.-% der Cannabinoidverbindung enthält.

6. Cannabinoidbeladene Granulate nach einem der Ansprüche 1 bis 5, wobei die Granulate Zucker sind, der mit einem Zuckersirup und einem Extrakt beschichtet ist, das mindestens eine Cannabinoidverbindung enthält.

7. Cannabinoidbeladene Granulate nach einem der Ansprüche 1 bis 6, wobei jedes cannabinoidbeladene Granulum eine Dosis von mindestens einer Cannabinoidverbindung von 2 mg bis 8 mg, plus oder minus 10%, enthält und vorzugsweise eine Dosis von gleich 5 mg, plus oder minus 10%, enthält.

8. Cannabinoidbeladene Granulate nach einem der Ansprüche 1 bis 7, wobei die Granulate ferner ein Terpen, vorzugsweise eine Kombination von mindestens zwei Terpenen, enthalten.

9. Cannabinoidbeladene Granulate nach einem der Ansprüche 1 bis 8, wobei die Granulate ferner beschichtet sind mit: einem Zuckersirup, der einen Farb- oder Aromastoff, natürliches Gummi, natürliches Wachs oder eine beliebige Kombination davon umfasst.

10. Verfahren zur Herstellung von cannabinoidbeladenen Granulaten nach einem der Ansprüche 1 bis 9, das mindestens die folgenden Schritte umfasst:
a) Beimengen mindestens einer Cannabinoidverbindung zu orodispersiblen Zuckergranulaten, die einen Durchmesser von 2 bis 10 Millimetern aufweisen,
b) Lufttrocknen der Granulate,
c) mindestens zwanzigfaches Wiederholen der aufeinanderfolgenden Schritte (a) und (b),
d) wahlweise Beschichten der cannabinoidbeladenen Granulate mit: einem Zuckersirup, der einen Farb- oder einen Aromastoff, natürliches Gummi, natürliches Wachs oder eine beliebige Kombination davon umfasst.

11. Cannabinoidbeladene Granulate nach einem der Ansprüche 1 bis 9, oder die nach einem der Verfahren nach Anspruch 10 erhalten wurden, zu ihrer Verwendung als ein Arzneimittel.

12. Cannabinoidbeladene Granulate nach einem der Ansprüche 1 bis 9, oder die nach einem der Verfahren nach Anspruch 10 erhalten wurden, zu ihrer Verwendung bei der Behandlung und/oder Prävention von chronischem Schmerz, Entzündungserkrankungen, Verhaltensstörungen und Angststörungen.

13. Verwendung von cannabinoidbeladenen Granulaten nach einem der Ansprüche 1 bis 9, oder die nach einem der Verfahren nach Anspruch 10 erhalten wurden, als Nahrungsergänzung.

14. Kit zur Verwendung als eine Nahrungsergänzung, das in einer Einzelverpackung mindestens zwei Abgabevorrichtungen umfasst, die cannabinoidbeladene Granulate umfassen, die ferner mindestens ein Terpen nach Anspruch 8 enthalten, wobei die mindestens zwei Abgabevorrichtungen sich darin voneinander unterscheiden, dass das mindestens eine Terpen sich von einer Abgabevorrichtung zur anderen unterscheidet.

## Revendications

1. Granules chargés de cannabinoïde consistant en des granules de sucre orodispersibles chargés d'au moins un composé cannabinoïde, **caractérisés en ce que** chaque granule présente un diamètre de 2 à 10 millimètres avant le chargement,
et **en ce que** les granules chargés de cannabinoïde sont destinés à être administrés par voie sublinguale.

2. Granules chargés de cannabinoïde selon la revendication 1, **caractérisés en ce que** lesdits granules de sucre orodispersibles sont composés de lactose, de saccharose, de xylitol, ou d'un mélange de ceux-ci.

3. Granules chargés de cannabinoïde selon la revendication 1 ou 2, **caractérisés en ce que** chaque granule présente un diamètre de 3 à 4 millimètres, plus ou moins 10 %, et/ou pèse 30 à 150 milligrammes, plus ou moins 10 %.

4. Granules chargés de cannabinoïde selon l'une quelconque des revendications 1 à 3, **caractérisés en ce que** ledit au moins un composé cannabinoïde est choisi dans le groupe constitué par : le cannabidiol (CBD), le cannabigérol, le trans-D9-tétrahydrocannabidiol (THC), et un mélange de ceux-ci.

5. Granules chargés de cannabinoïde selon l'une quelconque des revendications 1 à 4, **caractérisés en ce que** ledit au moins un composé cannabinoïde est synthétique, ou est extrait à partir de la plante *Cannabis sativa* et est sous la forme d'un isolat contenant au moins 95 % en poids dudit composé cannabinoïde, par rapport au poids total de l'isolat.

6. Granules chargés de cannabinoïde selon l'une quelconque des revendications 1 à 5, lesquels granules sont dragéifiés avec un sirop de sucre et un extrait contenant au moins un composé cannabinoïde.

7. Granules chargés de cannabinoïde selon l'une quelconque des revendications 1 à 6, **caractérisés en ce que** chaque granule chargé de cannabinoïde contient une dose d'au moins un composé cannabinoïde allant de 2 mg à 8 mg, plus ou moins 10 %, et de préférence contient une dose égale à 5 mg, plus ou moins 10 %.

8. Granules chargés de cannabinoïde selon l'une quelconque des revendications 1 à 7, lesdits granules contenant en outre au moins un terpène, de préférence une combinaison d'au moins deux terpènes.

9. Granules chargés de cannabinoïde selon l'une quelconque des revendications 1 à 8, lesdits granules étant en outre enrobés par : un sirop de sucre comprenant un agent colorant ou aromatisant, une gomme naturelle, une cire naturelle ou l'une quelconque de leurs combinaisons.

10. Procédé de production de granules chargés de cannabinoïde selon l'une quelconque des revendications 1 à 9, comprenant au moins les étapes suivantes :
a) addition d'au moins un composé cannabinoïde à des granules de sucre orodispersibles présentant un diamètre de 2 à 10 millimètres,
b) séchage à l'air des granules,
c) répétition desdites étapes successives (a) et (b) au moins vingt fois,
d) éventuellement enrobage des granules chargés de cannabinoïde par : un sirop de sucre comprenant un agent colorant ou aromatisant, une gomme naturelle, une cire naturelle, ou l'une quelconque de leurs combinaisons.

11. Granules chargés de cannabinoïde selon l'une quelconque des revendications 1 à 9, ou obtenus conformément au procédé de la revendication 10, pour leur utilisation en tant que médicament.

12. Granules chargés de cannabinoïde selon l'une quelconque des revendications 1 à 9, ou obtenus conformément au procédé de la revendications 10, pour leur utilisation dans le traitement et/ou la prévention d'une douleur chronique, de troubles inflammatoires, de troubles comportementaux, et de troubles anxieux.

13. Utilisation de granules chargés de cannabinoïde selon l'une quelconque des revendications 1 à 9, ou obtenus conformément au procédé de la revendication 10, en tant que complément nutritionnel.

14. Trousse pour son utilisation en tant que complément nutritionnel, comprenant, dans un seul emballage, au moins deux dispositifs distributeurs comprenant des granules chargés de cannabinoïde contenant en outre au moins un terpène selon la revendication 8, dans laquelle les au moins deux dispositifs distributeurs sont distincts l'un de l'autre en ce sens que ledit au moins un terpène est différent entre un dispositif distributeur et l'autre.
